# EUROPEAN PATENT SPECIFICATION

(11) **EP 2 165 078 B1**
(45) Date of publication and mention of the grant of the patent: **27.10.2010**
(21) Application number: 08762954.9
(22) Date of filing: 24.06.2008
(51) Int. Cl.: F04D 25/08, H02K 9/06

(54) **APPARATUS FOR REGULATED DELIVERY OF A GAS, IN PARTICULAR BREATHING APPARATUS**
VORRICHTUNG FÜR GEREGELTE GASABGABE, IM BESONDEREN FÜR EIN ATEMGERÄT
APPAREILS PERMETTANT UNE ALIMENTATION RÉGULÉE DE GAZ, APPAREILS RESPIRATEURS EN PARTICULIER

(30) Priority: 25.06.2007 US 937076 P; 03.07.2007 FR 0704780
(43) Date of publication of application: 24.03.2010
(73) Proprietor: Airfan, 31770 Colomiers (FR)
(72) Inventor: GRASMUCK, Gilbert, F-31880 La Salvetat Saint Gilles (FR)
(74) Representative: Junca, Eric
(86) International application number: PCT/IB2008/001644
(87) International publication number: WO 2009/001198

(56) References cited:
- EP-A- 1 100 184
- WO-A-00/17524
- WO-A-03/044364
- DE-A1- 3 710 622
- DE-A1- 10 200 951
- US-A1- 2006 073 030

## Description

The present invention relates to an apparatus for regulated delivery of a gas, in particular a breathing apparatus.

Document DE 37 10 622, which is considered as the closest prior art to the subject-matter of claim 1, teaches how to prepare a breathing apparatus comprising an air intake duct, a volute of which the inlet opening communicates with the air intake duct, a first vane rotor, located immediately downstream of the inlet opening of the volute, comprising an inlet opening connected to this inlet opening of the volute and outlet orifices opening into the volute, and a motor for rotating the rotor in order to generate a centrifugal air flow in the volute; this apparatus further comprises:
- a casing having a tubular portion of which the wall is intended to extend, after assembly, around the motor and remaining at a substantially constant distance from this motor, the casing and the motor thereby together bounding a gas flow passage comprising at least one gas inlet orifice and at least one gas outlet orifice; and
- a second vane rotor located downstream of said gas inlet orifice, rotated in order to generate a forced gas stream in said gas flow passage.

The shaft of the motor is mounted on ball bearings.

This existing apparatus has the prohibitive drawback of incurring a risk, in case of destruction of one or the other of the ball bearings, of an ejection of the balls from this bearing into the volute and therefore into the circuit of air delivered to the patient.

The existing apparatus also has the drawback of permitting a direct exchange of the gas fed to the volute with the motor compartment, so that the cooling of the motor depends on the gas flow rate in the volute.

Furthermore, the lifespan of the ball bearings of the vane rotors of this apparatus appears to be reduced.

It is the object of the present invention to remedy all these drawbacks.

The apparatus that it relates to comprises the abovementioned known features.

According to the invention, the apparatus comprises a plate traversed by the shaft of the motor, extending close to the wall of said first vane rotor opposite the inlet of the volute, and the casing comprises orifices causing communication between the side of this plate opposite said first vane rotor and said gas flow passage.

Thus, according to the invention, in case of destruction of one or the other of the shaft ball bearings, the plate serves to eliminate any risk of the introduction of the balls of this bearing into the gas delivery circuit.

Moreover, the plate serves to bound two essentially separate circuits, that is the main gas delivery circuit, comprising said first vane rotor and the volute, and the motor coolant gas circuit. Optimal cooling of the motor can be obtained independently of the gas flow rate in the main circuit, including when this flow rate is zero.

Furthermore, said orifices of the casing permit the flow, in said passage, of the pressurized gas flowing through the interstice necessarily existing between the shaft bearing said first vane rotor and the plate, and this gas leakage is collected by the motor coolant gas circuit and is removed with it. This leakage gas stream promotes the cooling of the part of the motor closest to said first vane rotor.

A further advantage of the invention is, thanks to said plate, in permitting the establishment of environmental conditions of the two ball bearings that are favorable to the lifespan of these bearings: the plate serves to create a slight pressure difference between the two sides of the motor and therefore to minimize the flushing of gas and any dust when passing through the bearings.

Preferably, said second vane rotor is placed on a shaft driven by the motor.

Thus, the speeds of rotation of the two vane rotors are identical, so that the speed of rotation of the second vane rotor and hence the cooling of the motor, can be adapted to the motor cooling requirements corresponding to the speed of rotation of said first vane rotor.

The apparatus also has a simple structure and, since said second vane rotor only generates a slight pressure, its power consumption is low and does not significantly deteriorate the energy balance of the operation of the apparatus.

Preferably, the casing comprises a plurality of gas outlet holes distributed on the circumference of this casing, and the casing comprises a circular chamber arranged at these gas outlet holes comprising a gas outlet manifold.

This circular chamber serves to collect the coolant gas, which can be reused. In fact, the temperature rise of this gas remains slight, about 10°C in extreme conditions; hence the flow of this gas can be used for example to cool other members, in particular sensitive components, such as electronic circuit boards or batteries for self-contained operation of the apparatus.

Advantageously, said circular chamber forms a body with the casing, at least in part.

The ventilation gas is preferably air, said gas inlet orifice opening directly onto the exterior of the casing.

This orifice allows an entry of air into said flow passage without disturbing this flow and without generating any noise.

The orifices of the casing causing communication between the side of the plate opposite said first vane rotor and said coolant gas flow passage are advantageously arranged at distant locations, in the radial direction, of the shaft of the motor.

This distancing serves to maximize the path of the leakage gas stream between the plate and the wall comprising said orifices, and thereby to promote the cooling of the motor on the side of said first vane rotor. Moreover, said orifices are thereby brought closer to the coolant gas outlet holes outside the casing, favoring the flow of this leakage stream.

The invention will be well understood, and other features and advantages thereof will appear, with reference to the appended schematic drawing, which shows, as a nonlimiting example, one possible embodiment of the apparatus that it concerns.
Figure 1 is a view of this apparatus, in an axial section along the axis of the shaft which this apparatus comprises and in a plane substantially parallel to the axis of the outlet manifold which the volute of this apparatus comprises, and
Figure 2 is a detailed view, at a larger scale.

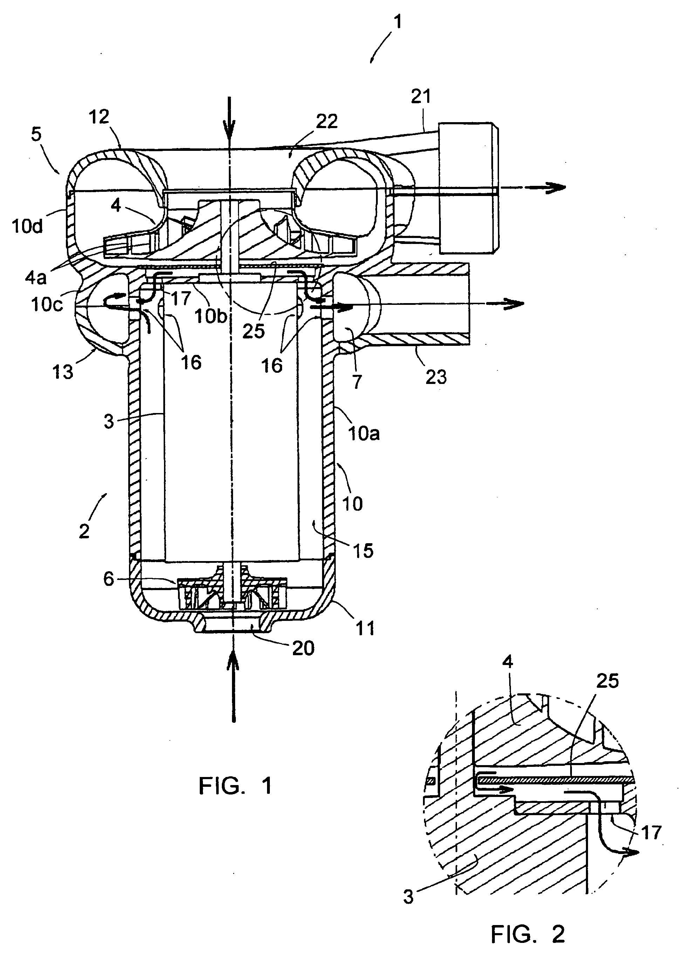

Figure 1 shows an apparatus 1 for regulated delivery of a gas, in particular a breathing apparatus, comprising a casing 2, a motor 3, a first vane rotor 4, a volute 5, a second vane rotor 6 and a collecting chamber 7.

The casing 2 comprises a main part 10, two end covers 11, 12 and a median cover 13.

The main part 10 forms a tubular portion 10a, for accommodating the motor 3, a transverse wall 10b bounding one longitudinal end of this tubular portion 10a, a circular wall 10c extending around this longitudinal end of the tubular portion 10a, and a circular wall 10d which forms the base of the volute 5.

The wall of the tubular portion 10a extends, after assembly, around the motor 3 and at a substantially constant distance from this motor, so that this tubular portion 10a and the motor 3 together bound an air flow passage 15.

This tubular portion 10a comprises, substantially at the height of the free edge of the circular wall 10c, a series of radial holes 16, and the transverse wall 10b comprises, at its periphery, a series of orifices 17 passing through it. These orifices 17 are arranged at distant locations, in the radial direction, of the shaft of the motor 3, and are therefore close to the holes 16.

The end cover 11 is intended to be placed on the free edge of the tubular portion 10a opposite the volute 5. It comprises an axial opening 20 facing the rotor 6 after assembly.

The end cover 12 comprises a part having a circular or spiral shape, intended to be placed on the free edge of the wall 10d, to constitute, with this wall 10d, the volute 5, and a part intended to constitute, with a corresponding part of the wall 10d, a tangential outlet manifold 21. This cover 12 forms an inlet opening 22 facing the rotor 4 after assembly, this opening 22 being in communication with an air intake duct (not shown) bounded by a casing (not shown) containing the apparatus 1.

The median cover 13 comprises a circular part intended to be placed on the free edge of the wall 10c, to constitute, with this wall 10c, the collection chamber 7, and a part intended to constitute, with a corresponding part of the wall 10c, an outlet manifold 23.

The motor 3 has a cylindrical shape and is axially connected to the transverse wall 10b, by means of screws (not shown). It comprises an outlet shaft projecting beyond its two axial ends, one of the projecting parts of this shaft accommodating the rotor 4 and the other projecting part accommodating the rotor 6.

This shaft is mounted on ball bearings, located respectively close to the rotor 4 and the rotor 6.

The rotor 4 comprises a hub, an upper flange and a bottom wall forming an air flow duct in which the vanes 4a of this rotor 4 extend. The upper flange forms a raised circular ledge immediately approaching the wall of the cover 12 bounding the opening 22.

The rotation of the rotor 4 at high speed, driven by the motor 3, generates a centrifugal air flow of which the kinetic energy is converted to pressure in the volute 5, this air entering via the opening 22 and exiting via the manifold 21.

The apparatus 1 further comprises a plate 25 traversed by the shaft of the motor 3, which extends close to the bottom wall of the rotor 4, this plate bearing against a circular seat arranged in the end of the tubular portion 10a and being attached thereto. The orifices 17 cause the side of this plate 25 opposite the rotor 4 to communicate with the passage 15.

As may be understood, the rotation of the rotor 6 by the motor 3 serves to generate a forced air stream in the passage 15, and then through the holes 16, into the chamber 7 and into the manifold 23. The passage 15 extends over the entire circumference of the motor 3 and has a continuous configuration, without a substantial variation in cross section, thereby serving to cool the motor 3 under ideal conditions. The air flow cooling the motor 3 is independent of the gas flow in the gas delivery circuit, so that the use of the apparatus has no effect on the cooling of the motor. This cooling is thus sufficiently effective even if the flow of the gas delivery circuit is zero.

The direct assembly of the rotors 4 and 6 on the same shaft of the motor 3 allows the speeds of rotation of the two vane rotors 4, 6 to be identical, thereby allowing the permanent adjustment of the cooling of the motor 3 to the speed of rotation of the rotor 4, hence to the operating speed of the apparatus 1.

The apparatus 1 also has a simple structure and, since the rotor 6 only generates a slight pressure, its power consumption is low and does not significantly deteriorate the energy balance of the operation of this apparatus.

The collection chamber 7 serves to collect the cooling air so that it can be reused, for example, to cool other members, in particular sensitive components such as electronic circuit boards or batteries for self-contained operation of this apparatus.

The plate 25, in case of destruction of one or the other of the ball bearings of the motor shaft, serves to eliminate any risk of the introduction of the balls of this bearing into the gas delivery circuit.

This plate 25 also serves to limit the leakage of pressurized gas contained in the volute 5, which occurs via the interstice that necessarily exists between this plate and the shaft bearing the rotor 4 (cf. Figure 2). The orifices 17 allow the flow of this leak into the passage 15, this leak being collected by the cooling air circuit of the motor 3 and removed therewith. Moreover, the leakage gas stream promotes the cooling of the part of the motor 3 closest to the rotor 4. This arrangement serves to establish, thanks to said plate 25, environmental conditions of the two ball bearings that are favorable to the lifespan of these bearings: the plate 25 serves to allow a slight pressure difference to exist between the two sides of the motor 3 and therefore to minimize the flushing of gas and any dust on passing through the bearings.

The apparatus according to the invention thus has decisive advantages over similar apparatus of the prior art.

The invention has been described above with reference to an embodiment provided purely as an example. It goes without saying that it is not limited to this embodiment but extends to all embodiments covered by the claims appended hereto.

## Claims

1. An apparatus (1) for regulated delivery of a gas, in particular breathing apparatus, comprising an air intake duct, a volute (5) of which the inlet opening (22) communicates with the air intake duct, a first vane rotor (4), located immediately downstream of the inlet opening (22) of the volute (5), comprising an inlet opening connected to this inlet opening (22) of the volute (5) and outlet orifices opening into the volute (5), and a motor (3) for rotating the rotor (4) in order to generate a centrifugal air flow in the volute (5), comprising:
- a casing (2) having a tubular portion (10a) of which the wall is intended to extend, after assembly, around the motor (3) and remaining at a substantially constant distance from this motor (3), the casing (2) and the motor (3) thereby together bounding a gas flow passage (15) comprising at least one gas inlet orifice (20) and at least one gas outlet hole (16); and
- a second vane rotor (6) located downstream of said gas inlet orifice (20), rotated in order to generate a forced gas stream in said gas flow passage (15);
**characterized in that** it comprises a plate (25) traversed by the shaft of the motor (3), extending close to the wall of said first vane rotor (4) opposite the inlet (22) of the volute (5), and **in that** the casing (2) comprises orifices (17) causing communication between the side of this plate (25) opposite said first vane rotor (4) and said gas flow passage (15).

2. The apparatus (1) as claimed in claim 1, **characterized in that** said second vane rotor (6) is placed on a shaft driven by the motor (3).

3. The apparatus (1) as claimed in either of claims 1 and 2, **characterized in that** the casing (2) comprises a plurality of gas outlet holes (16) distributed on the circumference of this casing (2), and **in that** the casing (2) comprises a circular chamber (7) arranged at these gas outlet holes (16) comprising a gas outlet manifold (23).

4. The apparatus (1) as claimed in claim 3, **characterized in that** said circular chamber (7) forms a body with the casing (2).

5. The apparatus (1) as claimed in one of claims 1 to 4, **characterized in that** said gas inlet orifice (20) opens directly onto the exterior of the casing (2).

6. The apparatus (1) as claimed in one of claims 1 to 5, **characterized in that** the ventilation gas is air, said gas inlet orifice (20) opening directly onto the exterior of the casing (2).

7. The apparatus (1) as claimed in one of claims 1 to 6, **characterized in that** the orifices (17) of the casing (2) causing communication between the side of the plate (25) opposite said first vane rotor (4) and said coolant gas flow passage (15) are arranged at distant locations, in the radial direction, of the shaft of the motor (3).

## Patentansprüche

1. Vorrichtung (1) zur geregelten Abgabe eines Gases, insbesondere Atemgerät, mit einem Lufteinlasskanal, einem Diffusor (5), dessen Einlassöffnung (22) mit dem Lufteinlasskanal in Verbindung steht, einem ersten Flügelrotor (4), der unmittelbar stromabwärts der Einlassöffnung (22) des Diffusors (5) angeordnet ist und eine Einlassöffnung, die mit dieser Einlassöffnung (22) des Diffusors (5) verbunden ist, und Auslassdurchtritte, die in den Diffusor (5) münden, enthält, und einem Motor (3) zum Drehen des Rotors (4) zur Erzeugung eines zentrifugalen Luftstroms im Diffusor (5), umfassend:
- ein Gehäuse (2) mit einem röhrenförmigen Teil (10a), von dem sich die Wand nach der Montage um den Motor (3) erstrecken und in einem im Wesentlichen konstanten Abstand von diesem Motor (3) bleiben soll, wobei das Gehäuse (2) und der Motor (3) somit zwischen sich einen Gasströmungsdurchgang (15) begrenzen, der mindestens einen Gaseinlassdurchtritt (20) und mindestens einen Gasauslassdurchlass (16) aufweist; und
- einen zweiten Flügelrotor (6), der stromabwärts des Gaseinlassdurchtritts (20) angeordnet ist und zur Erzeugung eines Zwangsgasstroms in dem Gasströmungsdurchgang (15) gedreht wird;
**dadurch gekennzeichnet, dass** sie eine Platte (25) umfasst, die von der Welle des Motors (3) durchquert wird und sich nahe der Wand des ersten Flügelrotors (4) gegenüber dem Einlass (22) des Diffusors (5) erstreckt, und dass das Gehäuse (2) Durchtritte (17) umfasst, die eine Verbindung zwischen der Seite dieser Platte (25), die dem ersten Flügelrotor (4) gegenüberliegt, und dem Gasströmungsdurchgang (15) herstellen.

2. Vorrichtung (1) nach Anspruch 1, **dadurch gekennzeichnet, dass** der zweite Flügelrotor (6) auf einer von dem Motor (3) angetriebenen Welle platziert ist.

3. Vorrichtung (1) nach Anspruch 1 oder 2, **dadurch gekennzeichnet, dass** das Gehäuse (2) mehrere Gasauslassdurchlässe (16) umfasst, die auf dem Umfang dieses Gehäuses (2) verteilt sind, und dass das Gehäuse (2) eine kreisförmige Kammer (7) umfasst, die an diesen Gasauslassdurchlässen (16) angeordnet ist und einen Gasauslassverteiler (23) umfasst.

4. Vorrichtung (1) nach Anspruch 3, **dadurch gekennzeichnet, dass** die kreisförmige Kammer (7) einstückig mit dem Gehäuse (2) ausgebildet ist.

5. Vorrichtung (1) nach einem der Ansprüche 1 bis 4, **dadurch gekennzeichnet, dass** der Gaseinlassdurchtritt (20) direkt in der Außenseite des Gehäuses (2) mündet.

6. Vorrichtung (1) nach einem der Ansprüche 1 bis 5, **dadurch gekennzeichnet, dass** das Lüftungsgas Luft ist, wobei der Gaseinlassdurchtritt (20) direkt in der Außenseite des Gehäuses (2) mündet.

7. Vorrichtung (1) nach einem der Ansprüche 1 bis 6, **dadurch gekennzeichnet, dass** die Durchtritte (17) des Gehäuses (2), die eine Verbindung zwischen der Seite der Platte (25), die dem ersten Flügelrotor (4) gegenüberliegt, und dem Kühlmittelgasströmungsdurchgang (15) herstellen, an in Radialrichtung entfernten Stellen der Welle des Motors (3) angeordnet sind.

## Revendications

1. Appareil (1) de délivrance régulée d'un gaz, notamment appareil d'assistance respiratoire, comprenant un conduit d'amenée d'air, une volute (5) dont l'ouverture d'entrée (22) est en communication avec le conduit d'amenée d'air, une première roue à ailettes (4), située immédiatement en aval de l'ouverture d'entrée (22) de la volute (5), comprenant une ouverture d'entrée reliée à cette ouverture d'entrée (22) de la volute (5) et des orifices de sortie débouchant dans la volute (5), et un moteur (3) d'entraînement de la roue (4) en rotation de manière à générer un flux d'air centrifuge dans la volute (5), comprenant :
- un boîtier (2) présentant une partie tubulaire (10a) dont la paroi est destinée à s'étendre, après montage, autour du moteur (3) en se trouvant à une distance sensiblement constante de ce moteur (3), le boîtier (2) et le moteur (3) délimitant ainsi entre eux un passage (15) d'écoulement de gaz comprenant au moins un orifice (20) d'entrée de gaz et au moins un trou (16) de sortie de gaz ; et
- une deuxième roue à ailettes (6) située en aval dudit orifice (20) d'entrée de gaz, entraînée en rotation de manière à générer un courant de gaz forcé dans ledit passage (15) d'écoulement de gaz ;
**caractérisé en ce qu'**il comprend une plaquette (25) traversée par l'arbre du moteur (3), s'étendant à proximité de la paroi de ladite première roue à ailettes (4) opposée à l'entrée (22) de la volute (5), et **en ce que** le boîtier (2) comprend des orifices (17) mettant en communication le côté de cette plaquette (25) opposé à ladite première roue à ailettes (4) et ledit passage (15) d'écoulement de gaz.

2. Appareil (1) selon la revendication 1, **caractérisé en ce que** ladite deuxième roue à ailettes (6) est placée sur un arbre entraîné par le moteur (3).

3. Appareil (1) selon la revendication 1 ou la revendication 2, **caractérisé en ce que** le boîtier (2) comprend une pluralité de trous (16) de sortie de gaz répartis sur la circonférence de ce boîtier (2), et **en ce que** le boîtier (2) comprend une chambre circulaire (7) aménagée au niveau de ces trous (16) de sortie de gaz, comportant une tubulure (23) de sortie de gaz.

4. Appareil (1) selon la revendication 3, **caractérisé en ce que** ladite chambre circulaire (7) forme corps avec le boîtier (2).

5. Appareil (1) selon l'une des revendications 1 à 4, **caractérisé en ce que** ledit orifice (20) d'entrée de gaz débouche directement sur l'extérieur du boîtier (2).

6. Appareil (1) selon l'une des revendications 1 à 5, **caractérisé en ce que** le gaz de ventilation est de l'air, ledit orifice (20) d'entrée de gaz débouchant directement sur l'extérieur du boîtier (2).

7. Appareil (1) selon l'une des revendications 1 à 6, **caractérisé en ce que** les orifices (17) du boîtier (2) mettant en communication le côté de la plaquette (25) opposé à ladite première roue à ailettes (4) et ledit passage (15) d'écoulement de gaz de refroidissement sont aménagés en des emplacements éloignés, dans le sens radial, de l'arbre du moteur (3).
